(19) Europäisches Patentamt
European Patent Office
Office européen des brevets

(11) Publication number: **0 576 447 B1**

(12) # EUROPEAN PATENT SPECIFICATION

(45) Date of publication of patent specification: **23.11.94**

(51) Int. Cl.⁵: **C07F 9/165**, B01F 7/16, C07C 333/16

(21) Application number: **92905537.4**

(22) Date of filing: **04.03.92**

(86) International application number: **PCT/EP92/00493**

(87) International publication number: **WO 92/15595 (17.09.92 92/24)**

(54) **APPARATUS FOR THE PRODUCTION OF PHOSPHOROUS OR NITROGEN-CONTAINING COMPOUNDS.**

(30) Priority: **05.03.91 GB 9104612**

(43) Date of publication of application: **05.01.94 Bulletin 94/01**

(45) Publication of the grant of the patent: **23.11.94 Bulletin 94/47**

(84) Designated Contracting States: **BE DE ES FR GB IT NL**

(56) References cited:
**FR-A- 1 028 809**
**US-A- 2 862 947**
**US-A- 2 882 149**
**US-A-30 868 49**

(73) Proprietor: **EXXON CHEMICAL PATENTS INC.**
**200 Park Avenue**
**Florham Park New Jersey 07932 (US)**

(72) Inventor: **LOMBARDI, Alessandro**
**Via Lagustena, 136/23**
**I-16131 Genova (IT)**
Inventor: **BARINI, Geraldo**
**Via Paganini, 1**
**I-57100 Livorno (IT)**
Inventor: **CHIERICI, Enrico**
**Via S. Chiesa di Fontanegli, 23**
**I-16165 Genova (IT)**
Inventor: **D'ANTONIO, Carmine**
**Via Gastaldi, 34**
**I-17042 Bereggi (IT)**
Inventor: **GUSI, Stefano**
**Via Allende, 16**
**I-40139 Bologna (IT)**

(74) Representative: **Northover, Robert Frank et al**
**Exxon Chemical Limited**
**Exxon Chemical Technology Centre**
**PO Box 1**
**Abingdon Oxfordshire, OX13 6BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to improvements in the manufacture of phosphorus- or nitrogen-containing compounds, especially compounds suitable for use as lubricating oil additives or as intermediates suitable for use in the manufacture of such additives.

For the past 30 to 50 years, lubricating oils used as crankcase lubricants in internal combustion engines in automobiles and trucks have normally contained phosphorus- or nitrogen-containing compounds, which in many cases also contained sulphur, to improve their efficiency and useful life. One important class of such compounds comprises zinc dihydrocarbyl dithiophosphates (ZDDPs). ZDDPs are primarily antiwear agents, but also provide antioxidant activity. Another important class of such compounds comprises metal dithiocarbamates (DTCs), which may be included in lubricating oil to provide a source of, for example, sulphur.

In view of the very considerable commercial importance of ZDDPs and DTCs, many different proposals have been made over many years for improving processes for their production, examples of such proposals being those described in British Patent Specification No. 2 194 239A and East German Patent Specification No. 278 799A. US-A-2862947 and US-A-3086849 disclose a continuous process said to be applicable to the preparation of dithiophosphate acid esters, in which a circulating slurry containing phosphorus sulphide and dithiophosphate acid ester is agitated while introducing excess phosphorous sulphide and alcohol. Despite the very considerable work that has been carried out in this field, however, the applicants have found that it is possible, in a relatively simple manner, to make very significant improvements in the production of these and related compounds.

The invention provides a process for the manufacture of a compound of the general formula

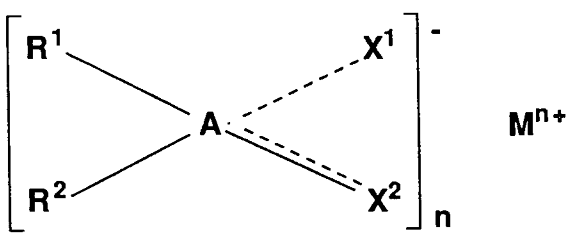

(I)

wherein A represents an -O-P-O- or an >N-C group;
each of $X^1$ and $X^2$, which may be the same or different, represents O or S;
each of $R^1$ and $R^2$, which may be the same or different, represents a hydrocarbyl radical,
M represents H, a metal ion, or an ammonium group; and
n represents an integer equal to the valency of M,
with the proviso that, when A represents an >N-C group, both $X^1$ and $X^2$ represent S,
in which process a first reactant is present in a different phase from a second reactant and the said reactants are reacted in a region defined by a surface and at least one member adjacent to the surface, there being relative movement between the surface and the member(s) such that the reactants are caused to move relative to the surface. Advantageously, the region is defined by adjacent surfaces between which there is relative movement such that the reactants are caused to move relative to one or both of the surfaces.

The invention also provides a process for the manufacture of a compound of the general formula

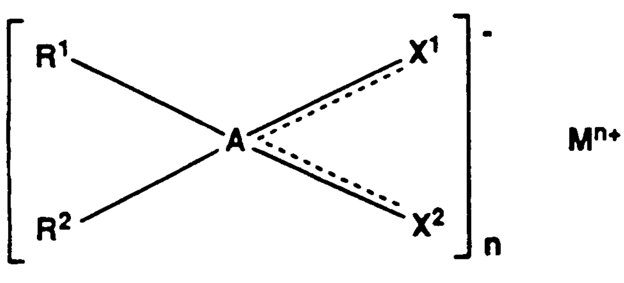

(I)

wherein A represents an -O-P-O- or an >N-C group;
each of $X^1$ and $X^2$, which may be the same or different, represents O or S;
each of $R^1$ and $R^2$, which may be the same or different, represents a hydrocarbyl radical,
M represents H, a metal ion, or an ammonium group; and
n represents an integer equal to the valency of M,
with the proviso that, when A represents an >N-C group, both $X^1$ and $X^2$ represents S,
in which process a first reactant is present in a different phase from a second reactant and the reactants are reacted in a thin film on a surface over which they move, the average velocity (taken across the thickness of the film) of a mixture of the reactants in a direction parallel to the surface being at least 1 m/sec. The thin film is advantageously formed between the surface and at least one member adjacent to the surface, preferably between adjacent surfaces, there being relative movement between the surface and the member-(s), or between the surfaces, causing the said movement of the reactants.

The invention also provides compounds of the above general formula prepared by the process of the invention, especially such compounds which are suitable for use as additives for lubricating oils.

The compounds of the general formula given above contain two hydrocarbyl radicals, which may be the same or different. As used in this specification, the term "hydrocarbyl" denotes a radical having a carbon atom directly attached to the remainder of the molecule and having a hydrocarbon or predominantly hydrocarbon character. Hydrocarbyl radicals include the following:

(1) Hydrocarbon groups; that is, aliphatic, (e.g. alkyl or alkenyl), alicyclic (e.g. cycloalkyl or cycloalkenyl), aromatic, aliphatic- and alicyclic-substituted aromatic, and aromatic-substituted aliphatic and alicyclic groups, and cyclic groups wherein the ring is completed through another portion of the molecule (that is, any two indicated substituents may together form an alicyclic group). Examples of hydrocarbon groups include methyl, ethyl, octyl, decyl, octadecyl, cyclohexyl, and phenyl groups.

(2) Substituted hydrocarbon groups; that is, groups containing non-hydrocarbon substituents which do not alter the predominantly hydrocarbon character of the group. Examples of suitable substituents include halo, hydroxy, nitro, cyano, alkoxy, and acyl groups.

(3) Hetero groups; that is, groups which, while predominantly hydrocarbon in character, contain atoms other than carbon in a chain or ring otherwise composed of carbon atoms. Suitable hetero atoms include, for example, nitrogen, oxygen and sulphur.

In general, no more than about three substituents or hetero atoms, and preferably no more than one, will be present for each 10 carbon atoms in the hydrocarbyl group.

Especially preferred compounds for use in oil-based compositions are those wherein $R^1$ and $R^2$ in the above general formula contain from 1 to 18, and preferably 2 to 12, carbon atoms. Particularly preferred as $R^1$ and $R^2$ radicals are alkyl radicals having 2 to 8 carbon atoms. Examples of radicals which $R^1$ and $R^2$ may represent are ethyl, n-propyl, i-propyl, n-butyl, i-butyl, sec-butyl, amyl, n-hexyl, i-hexyl, n-heptyl, n-octyl, decyl, dodecyl, octadecyl, 2-ethylhexyl, phenyl, butylphenyl, cyclohexyl, methylcyclopentyl, propenyl and butenyl radicals.

The total number of carbon atoms in $R^1$ and $R^2$ is preferably sufficient to impart oil-solubility to the compounds. In order to obtain oil solubility, the total number of carbon atoms in $R^1$ and $R^2$ will generally be about 5 or greater.

M in the above general formula represents hydrogen, a metal ion, or an ammonium group.

When M in the above general formula represents a metal ion, M is advantageously derived from a Group Ia metal, a Group IIa metal, aluminum, tin, lead, molybdenum, titanium, manganese, cobalt, nickel, copper, cadmium, antimony or zinc. For some uses, M preferably represents a zinc ion, as in ZDDPs. In other cases, compounds wherein M represents copper or molybdenum may have particular uses. Thus, for example, as indicated later in this specification, copper dihydrocarbyl dithiophosphates and copper dithiocarbamates have proved very useful as, inter alia, antioxidants, and molybdenum dithiocarbamates also have important uses.

An ammonium cation represented by M may be derived from ammonia or a primary, secondary or tertiary amine. The ammonium cation is preferably a cation of the formula $R^3R^4R^5R^6N^+$ wherein $R^3$, $R^4$, $R^5$ and $R^6$ each independently represents hydrogen, or a hydrocarbyl, aminohydrocarbyl, hydroxyhydrocarbyl, or hydroxyhydrocarbyloxyhydrocarbyl group, or $R^3$ and $R^4$ may be hydrocarbyl groups joined together to form a ring including the nitrogen atom, and optionally, oxygen, sulphur, or other nitrogen atoms. When the groups $R^3$, $R^4$, $R^5$ and/or $R^6$ represent hydrocarbyl groups, these groups are generally hydrocarbyl groups containing up to about 150 carbon atoms and will more often be aliphatic hydrocarbyl groups containing from about 4 to about 30 carbon atoms.

Compounds of the general formula given above which find particular use a lubricating oil additives include compounds of the formula $[(R^1O)(R^2O)P(S)(O)]_n^- M^{n+}$, and, especially, compounds of the formula [-

$(R^1O)(R^2O)P(S)S]_n^- M^{n+}$ (particularly dihydrocarbyl dithiophosphates in which M represents Zn or Cu) and compounds of the formula $[R^1R^2NC(S)S]_n^- M^{n+}$ (particularly dithiocarbamates in which M represents Zn, Mo or Cu). Copper may be in the cuprous or cupric form.

The invention is applicable to processes for manufacturing compounds of the general formula (I) involving a chemical reaction between reactants which are present in different phases, that is, reactants which are to some extent mutually insoluble. Normally, each of the first and second reactants is present in the reaction mixture as a liquid or solid.

ZDDPs are normally prepared by a first step which comprises forming a dihydrocarbyl dithiophosphoric acid (a DDPA), usually by reaction of one or more alcohols or phenols with a phosphorus sulphide, usually $P_2S_5$ (although $P_4S_3$ and other sulphides may be used), and a second step in which the dihydrocarbyl dithiophosphoric acid, and optionally an added carboxylic acid, is neutralised with a zinc compound, for example, zinc oxide, zinc hydroxide, or zinc carbonate. Analogous methods may be used for other metal dihydrocarbyl dithiophosphates, replacing the zinc compound by a compound of the metal in question.

Both the first step and the second step in the preparative method described above involve the reaction of a particulate solid (the $P_2S_5$ in the first step and the metal compound in the second step) with a liquid (the alcohol or phenol in the first step, and the DDPA in the second step). The solid is in each case to some extent insoluble in the liquid, that is, the liquid and the solid are present in different phases. The applicants have surprisingly found that very significant improvements in this long-established preparative method can be obtained if the first step, or the second step, or, preferably, both steps are carried out in accordance with the invention.

Where the first step is carried out in accordance with the invention, this step may be carried out in a batchwise or, where appropriate, continuous manner. Ambient pressures may be used if desired. The temperature may be, for example, in the range of from 50 to 150 °C. The process is particularly suitable for reacting phosphorus sulphides ranging from, for example, $P_4S_3$ to $P_4S_{12}$, with $C_3$ to $C_{20}$ primary or secondary aliphatic alcohols, and alkylated phenols. A variable amount of hydrogen sulphide is produced, the amount in any particular case depending on the nature of the starting phosphorus sulphide. Where the reaction is carried out in a batchwise manner, the reactants may, if desired, be caused to circulate through a reactor used in accordance with the invention, in which the reactants are reacted in a region between adjacent surfaces, and a conventional stirred tank reactor or other "soaking" zone.

Where the second step (the neutralisation step) is carried out in accordance with the invention, this step may be carried out batchwise or, where appropriate, continuously, and may if desired or required be promoted by, for example, the addition to the reaction mixture of small amounts of a carboxylic acid or a salt of such an acid. Alternatively, a mixed ZDDP/carboxylate salt may be prepared by the incorporation of relatively larger amounts of carboxylic acid in this step. Neutralisation may be carried out at ambient pressures. The temperature used may, for example, be in the range of from 50 to 150 °C, the temperature used in any particular case depending on the nature of the acid being neutralized.

The first step and/or the second step may if desired be carried out at reduced pressures to facilitate the removal of gaseous by-products.

In carrying out reactions in accordance with the invention, particularly advantageous results may be obtained if the mass ratio of the reactants is kept substantially constant during the reaction. The optimum ratio for any given apparatus and reaction can be ascertained by routine experiment. For guidance, particularly advantageous mass ratios of reactants for the specific processes referred to above carried out in one type of laboratory-scale reactor are indicated in Table 1, where

$$\text{\% heterogeneous phase} = \frac{\text{mass of reactant used in the smaller proportion}}{\text{mass of reactant used in the larger proportion}} \times 100$$

For the specific processes referred to above and in Table 1, the reactant present in the solid phase is the reactant used in the smaller proportion.

Table 1

| Reaction | % heterogeneous phase | |
|---|---|---|
| | Broad | Preferred |
| DDPA Production | 10-60 | 25-45 |
| Neutralisation | 0.5-20 | 1-12 |

The mass of the liquid phase reactant may in some cases include the mass of a solvent or diluent Examples of improvements that may be obtained in the production of ZDDPs by a process in accordance with the invention are one or more of improved DDPA and ZDDP quality, with fewer unwanted by-products, reduction in the risk of ZDDP decomposition, improved utilization of raw materials, improved rate of production of the product and/or a reduction in amounts of sediment obtained at the end of the process, with a consequent reduction in amounts of unwanted material to be removed, for example, by filtration, and to be disposed of. Indeed, in some cases, it may be possible completely to eliminate the need for a final filtration step. Further, in some cases, for example, when using primary alcohols, the invention may make it possible to carry out the process in a continuous manner, rather than batchwise. In certain cases, the use of a semicontinuous procedure, where one or more reactants are added while a reaction is already taking place, may be advantageous.

Examples of advantageous results which have been observed in laboratory preparations of ZDDPs, by a process in accordance with the invention carried out in a reactor of the type used in the Examples herein are a 10-fold reduction in final sediment or a 20 to 50-fold increase in rate of production, results obtained in accordance with the invention being compared in each case with results obtained when carrying out the same process in a conventional stirred reactor.

It will be appreciated that, although the above discussion is primarily in terms of ZDDPs, the invention is also applicable to the preparation of ammonium salts and other metal salts of dihydrocarbyl dithiophosphoric acids. Further, the first step indicated above may be used in the preparation of materials which are not subsequently reacted with zinc compounds to form ZDDPs, for example, in the reaction of $P_2S_5$ and alkyl-phenols to produce alkyl phenol dithiophosphoric acids for use, for example, as antioxidants. Dihydrocarbyl dithiophosphoric adics prepared in the first step may also be used as intermediates to prepare, for example, their sodium, potassium, magnesium, calcium, barium, nickel, copper and antimony salts, and salts of other metals as indicated above. Such salts, depending on the nature of the starting alcohol/phenol, may be used as antioxidant and/or antiwear agents for lubricants. DDPAs derived from alkyl phenols may also, for example, be reacted with unsaturated organic compounds, for example, styrene or methyl acrylate to produce ashless additives.

A process in accordance with the invention is advantageously carried out continuously, where it is appropriate to do so.

Metal and ammonium salts of a dihydrocarbyl monothiophosphoric acid may be prepared by reacting the acid with a metal compound or an amine. Alternatively, the metal or ammonium salts may be prepared, for example, by reacting a dihydrocarbylphosphite with a sulphur source in the presence of a metal compound or an amine. Examples of sulphur sources are elemental sulphur, sulphur halides, combinations of sulphur or sulphur oxides with hydrogen sulphide, and various sulphurized organic compounds. The preparation of dihydrocarbyl monothiophosphates is described in, for example, International Specification WO87/07638, the disclosures of which are incorporated by reference herein. Metal and ammonium salts of dihydrocarbyl phosphates may be prepared by reaction of the corresponding acid with a metal compound or an amine.

Dithiocarbamates may be prepared, for example, by reacting a secondary amine of the formula $R^1R^2NH$, wherein $R^1$ and $R^2$ have the meanings given above, with carbon disulphide and sodium hydroxide. The resulting sodium dithiocarbamates may then be treated, if desired, with a salt of another metal, for example, a zinc salt, to replace the sodium by the metal in question. Methods for the preparation of dithiocarbamates are well known in the art.

The process of the invention may be used where any method for the preparation of any of the compounds falling within the scope of general formula (I) involves the use of reactants that are to some extent mutually insoluble, that is, reactants in different phases.

The invention also provides the use of apparatus comprising a reaction region defined by a surface and at least one member adjacent to the surface, and means for causing relative movement between the surface and the member(s), for reducing sediments in a process for the manufacture of a compound of the

general formula (I) given above.

The invention further provides the use of apparatus comprising means for causing reactants to move in a thin film over a surface, for reducing sediments in a process for the manufacture of a compound of the general formula (I) given above.

In accordance with one aspect of the invention, reactants in different phases are reacted in a region defined by a surface and at least one member adjacent to the surface, there being relative movement between the surface and the member(s) such that the reactants are caused to move relative to the surface. Advantageously, the region is defined by adjacent surfaces between which there is relative movement such that the reactants are caused to move relative to one or both of the surfaces.

Because the surface and the member(s) (or, in the preferred case, the two surfaces) defining the reaction region are adjacent to each other, the reactants are reacted in a thin film and are caused to move relative to the surface or, if there are two surfaces, to at least one of the surfaces. The very high mechanical forces exerted on the reactants as a result of the arrangement and action of the surface/member(s) and the surfaces result in significant improvements as described elsewhere in this specification.

In a further aspect of the invention, the reactants are caused to move in a thin film on a surface over which they move, the average velocity (taken across the thickness of the film) of a mixture of the reactants in a direction parallel to the surface being at least 1 m/sec, the thin film advantageously being formed between the surface and at least one member adjacent to the surface, preferably between adjacent surfaces, there being relative movement between the surface and the member(s), or between the surfaces, causing the said movement of the reactants.

A thin film in accordance with the invention advantageously has a thickness of at most 25 mm, preferably at most 20 mm, and especially at most 15 mm. In particular applications, film thicknesses in the range of from 0.1 to 10 mm, especially 0.2 to 10 mm, have been found to be especially advantageous.

Although the invention extends to arrangements where this is not the case, the film thickness is advantageously determined by the width of the gap between the surface and at least one member adjacent to the surface (and preferably, between adjacent surfaces), and the said gap advantageously has a width of at most 25 mm, preferably at most 20 mm, and especially at most 15 mm. (In accordance with the invention, therefore, the surface and the member, or two surfaces, are said to be "adjacent" if they define a reaction region which advantageously has a thickness of at most 25 mm.) In particular forms of apparatus suitable for use in accordance with the invention, gaps with a width in the range of from 0.1 to 10 mm, especially 0.2 to 10 mm, have been found to be particularly advantageous. The width of the gap need not be constant throughout the reaction region.

Where there is a thin film of material between a surface and at least one member adjacent to the surface (preferably between adjacent surfaces), relative movement of the surface and the member(s) or between the surfaces causes movement of the mixture of reactants relative to the or a surface. In the preferred case, discussed in more detail below, where the surface, or one of the surfaces, is stationary and the member(s) or the other surface moves, the mixture will tend to move in a direction parallel to the stationary surface with an average velocity less than, but for a thin film approaching, that of the adjacent moving member/surface. Thus, for example, where the reactants are reacted in a thin film formed between an inner rotor and an outer stator, the mixture of reactants will tend to move over the surface of the stator with an average velocity less than, but approaching, the peripheral velocity of the rotor. In accordance with the invention, the mixture advantageously moves with an average velocity (taken across the thickness of the film) of at least 1 m/sec, advantageously at least 5 m/sec, and preferably at least 10 m/sec, and these are preferred peripheral velocities for the rotor in arrangements where the reactants are reacted in a region defined by an inner body and an adjacent outer housing, one of which, preferably the inner body, forms a rotor and the other of which forms a stator. In one particularly preferred arrangement having a central rotor and an outer stator, a peripheral speed of the rotor in the range of from 20 to 30 m/sec was found to be especially advantageous.

In an embodiment not at present preferred, both the surface and the member(s), or both the surfaces, move. If the surface and member(s), or the two surfaces, move in opposite directions (for example, if an inner body and an adjacent outing housing are rotated in opposite directions),the minimum average velocity of 1 m/sec mentioned above is the velocity relative to the surface or one of the surfaces.

It will be appreciated that the movement of the mixture of reactants will normally have components in more than one direction. Thus, for example, in an arrangement where the reactants are reacted in a region defined by an inner body and an adjacent outer housing, one of which rotates, the movement of the mixture will normally have components in the axial and radial directions as well as in the tangential direction. In such an arrangement, the minimum average velocity of 1 m/sec referred to herein is the component of velocity in the tangential direction. As indicated above, where the inner body and outer housing are adjacent

to each other so that there is a thin film of material between them, the mixture of reactants has an average velocity (taken across the thickness of the film) having a component in the tangential direction approaching the peripheral speed of the rotor.

When the reaction region is defined by a surface and at least one member adjacent to the surface (and, preferably, by two adjacent surfaces), the surface and/or the member(s) (or one or both of the surfaces) may be caused to move, although it is normally preferred that the or a surface remains stationary. Any type of movement may be employed. Thus, for example, the movement may be an oscillatory movement. Advantageously, however, the member(s) or, where there are two surfaces, one of the surfaces, rotates, the, or the other, surface remaining stationary.

Where the reaction region is defined by a surface and at least one member adjacent to the surface, the or each member may comprise, for example, a blade extending radially outwardly from a shaft which, in use, is rotated at high speed. Such rotation will cause the reactants to form a thin film on the surface and to move over the surface. There may also be at least one zone where centrifugal forces cause the reactants to travel in a generally radial direction at a considerable velocity before meeting the surface. In some cases, centrifugal forces may be sufficient to maintain on the surface a thin film whose thickness is less than the width of the gap between the surface and the outer extremities of the blades.

In the preferred case, where reaction of the reactants takes place in a region defined by two surfaces, the reaction region may, if desired, be defined by opposing surfaces of two plates or cylinder walls, for example, discs, although this arrangement is not at present preferred. Preferably one of the plates is rotated at high speed, for example, from 500 to 10,000 rpm, depending on the diameter, while the other plate is stationary. The opposing surfaces of the plates are relatively close to each other (that is, adjacent to each other) so that only a thin film of material to be reacted is present between them at any one time, the gap between the plates preferably being, for example, of the order of 0.2 to 25 mm. The plates may be, for example, discs having a diameter of up to 50 cm, although larger diameters are not excluded. Where one disc is rotated and the other is stationary, the peripheral velocity of the rotating disc is advantageously at least 1 m/sec, advantageous and preferred velocities being as indicated above in connection with a rotor/stator arrangement.

In a particular preferred embodiment of the invention, the reaction region is a generally annular region defined by a surface of an inner body and a surface of an outer housing, and the material in the region is caused to move by rotation of the inner body and/or the outer housing, advantageously by rotation of the inner body while the housing remains stationary.

References to a "generally annular" region include not only the case where the inner body is in the form of a right circular cylinder and the inner surface of the housing is also smooth, but also the case where one or both of the opposed surfaces defining the mixing region is not smooth. Thus, for example, one or both of the surfaces may have one or more protrusions thereon or one or more depressions therein (including the cases where there is a single helical protuberance and/or a single helical groove) or may be, for example, toothed or corrugated. For example, the outer housing may have a plurality of inwardly-extending protrusions thereon, the protrusions, which are preferably all of the same size and shape as one another, preferably being spaced apart at regular intervals on the inner surface of the outer housing, and the inner body (the rotor in this case) may have a plurality of outwardly extending regularly spaced projections of the same size and shape as one another. Alternatively, or in addition, the inner body or the housing may have one or more apertures or discontinuities (for example, slots) therein.

Although in the preferred arrangements described above, the outer diameter of the inner body (or, where the inner body is not in the form of a right circular cylinder, the diameter of the volume swept out by the inner body if it were to rotate) and the inner diameter of the housing (or the diameter of the volume that would be enclosed in the interior of the housing if it were to rotate) are advantageously constant along the length of the rotor, one or both of the diameters may vary along the length of the gap. Thus, for example, starting from one end of the inner body and housing, the inner diameter of the housing may remain constant or decrease, while the external diameter of the inner body increases, or both said diameters may increase but at different rates, or the external diameter of the inner body may remain constant while the internal diameter of the housing decreases.

In the arrangements discussed above, the inner body may be located coaxially or eccentrically with respect to the housing, but preferably the housing and inner body are radially symmetrical and coaxial.

As indicated earlier, the inner body and/or the outer housing may have one or more apertures or discontinuities therein. Such apertures or discontinuities enable material to pass through the body in question in a generally radial direction, so that, for example, reactants fed to the centre of a hollow rotor can travel radially outwards to react in the gap between the rotor and stator. Further, the stator may also, or alternatively, have apertures and or discontinuities therein. Where the rotor and/or stator has apertures or

discontinuities, it may be possible to use more than one rotor and/or more than one stator. Thus, for example, reactants may pass outwardly, in a generally radial direction, through a first stator, a rotor, and a second stator.

Apertures or discontinuities may of course be present in any body providing a surface referred to in this specification. Where such an annular body has a plurality of discontinuities in the form of slots therein, the body can, in the extreme case, be regarded as a plurality of members as discussed elsewhere in this specification.

Where materials pass through a body providing a surface they will in general be subjected to additional forces, for example, shearing forces, which may enhance the results obtainable in accordance with the invention. Shearing forces may be of importance in all aspects of the invention.

An especially advantageous reactor for use in accordance with the invention comprises an outer casing having within it one or more stators, which may be interconnected with each other, and one or more rotors, which may also be interconnected with each other, such that there is at least one reaction region defined by a rotor and an adjacent stator. Advantageously, the or each stator and the or each rotor in such a reactor describes at least a part of the curved wall of a right circular cylinder, the rotor(s) and stator(s) being coaxial with each other and, in the preferred case where the casing also has the general form of a right circular cylinder, with the casing.

Where there is more than one stator and/or more than one rotor, there will normally be more than one reaction region according to the invention. In the last-mentioned case, it will normally be desirable for one or more of the stator(s) and rotor(s) to have apertures and/or discontinuities therein so that the reactants can readily pass from one reaction region to another. Thus, for example, a rotor or stator may have a plurality of circular apertures therethrough, or may comprise a circumferentially continuous portion from which a plurality of wall portions extend in a generally axial direction to give a generally cylindrical surface with discontinuities therein.

An advantageous reactor as described above may be provided with one or more inlets and outlets such that reactants can be introduced in an axial direction to the centre of the rotor/stator arrangement and, after passing radially outwards through the rotor/stator arrangement, can be withdrawn from a zone between the said arrangement and the casing, or from a zone, for example, a post-reaction zone, downstream of the said zone. The rotor advantageously acts as a pump to draw reactants fed axially into the reactor to the centre of the rotor/stator arrangement, and also subjects the reactants to centrifugal forces causing them to move radially outwards through the rotor/stator arrangement. A reactor of this type is very suitable for use in carrying out reactions in a continuous manner. A rotor which causes or assists movement of fluids within the reactor may of course be used in reactors other than the especially advantageous reactor described above.

For any given reaction, the reaction time required to give optimum results will depend, inter alia, on the nature of the reactants and the reaction temperature, and can be ascertained by routine experiment. If the reaction time is too low, yields may be undesirably low, while if the reaction time is too high, unwanted reactions may occur.

Some reaction of the reactants used in accordance with the invention may occur other than in the reaction zone (the region/thin film) used in accordance with the invention. Thus, for example, the reactants may be mixed before being introduced into the said region/thin film, and some reaction may take place at that stage, or in a post-reaction zone (for example, a "soaking" zone) downstream of the reaction zone used in accordance with the invention. Whether or not reaction occurs elsewhere, advantageously a major part of each of the reactants is treated in a reaction zone used in accordance with the invention. Preferably at least 75 mass% of each of the reactants is treated in the said reaction zone, especially at least 85 mass%, and particularly at least 95 mass%. In the most preferred case, substantially all of each of the reactants is treated in the said reaction zone. Thus the arrangements of inlet(s) and outlet(s) in any given reactor, and the internal design of the reactor, are preferably such that, in passing through the reactor, the reactants are constrained to pass through the reaction zone.

The invention further provides the use of apparatus comprising an outer housing and a rotor within the housing, the housing and the rotor having opposed adjacent surfaces defining a reaction region, in the preparation of compounds of general formula (I) given above, especially such compounds which are suitable for use as additives for lubricating oils, the peripheral speed of the rotor preferably being at least 1 m/sec.

Lubricating oil additives prepared in accordance with the invention are oil-soluble or (in common with certain of the other additives referred to below) are dissolvable in oil with the aid of a suitable solvent, or are stably dispersible materials. Oil-soluble, dissolvable, or stably dispersible as that terminology is used herein does not necessarily indicate that the materials are soluble, dissolvable, miscible, or capable of being

suspended in oil in all proportions. It does mean, however, that the additives are, for instance, soluble or stably dispersible in oil to an extent sufficient to exert their intended effect in the environment in which the oil is employed. Moreover, the additional incorporation of other additives may also permit incorporation of higher levels of a particular additive, if desired.

Additives prepared in accordance with the present invention can be incorporated into the oil in any convenient way. Thus, they can be added directly to the oil by dispersing or by dissolving them in the oil at the desired level of concentration, typically with the aid of a suitable solvent such, for example, as toluene, cyclohexane, or tetrahydrofuran. Such blending can occur at room temperature or an elevated temperature.

Additives prepared in accordance with the invention are particularly useful in lubricating oil compositions which employ a base oil in which the mixtures are dissolved or dispersed. Base oils with which the additives may be used include those suitable for use as crankcase lubricating oils for spark-ignited and compression-ignited internal combustion engines, for example, automobile and truck engines, marine and railroad diesel engines.

Synthetic base oils include alkyl esters of dicarboxylic acids, polyglycols and alcohols; poly-$\alpha$-olefins, polybutenes, alkyl benzenes, organic esters of phosphoric acids and polysilicone oils.

Natural base oils include mineral lubricating oils which may vary widely as to their crude source, for example, as to whether they are paraffinic, naphthenic, mixed, or paraffinic-naphthenic, as well as to the method used in their production, for example, distillation range, straight run or cracked, hydrofined, solvent extracted and the like.

More specifically, natural lubricating oil base stocks which can be used may be straight mineral lubricating oil or distillates derived from paraffinic, naphthenic, asphaltic, or mixed base crude oils. Alternatively, if desired, various blended oils may be employed as well as residual oils, particularly those from which asphaltic constituents have been removed. The oils may be refined by any suitable method, for example, using acid, alkali, and/or clay or other agents such, for example, as aluminium chloride, or they may be extracted oils produced, for example, by solvent extraction with solvents, for example, phenol, sulphur dioxide, furfural, dichlorodiethyl ether, nitrobenzene, or crotonaldehyde.

The lubricating oil base stock conveniently has a viscosity of about 2.5 to about 12 cSt (about 2.5 x $10^{-6}$ to about 12 x $10^{-6}$ $m^2/s$) and preferably about 2.5 to about 9 cSt. (about 2.5 x $10^{-6}$ to about 9 x $10^{-6}$ $m^2/s$) at 100 °C.

An additive prepared in accordance with the present invention may be employed in a lubricating oil composition which comprises lubricating oil, typically in a major proportion, and the additive, typically in a minor proportion, for example, in a proportion as indicated below. Additional additives may be incorporated in the composition to enable it to meet particular requirements. Examples of additives which may be included in lubricating oil compositions are viscosity index improvers, corrosion inhibitors, oxidation inhibitors, friction modifiers, dispersants, metal-containing detergents, anti-foaming agents, anti-wear agents, pour point depressants, and rust inhibitors.

Viscosity index improvers (or viscosity modifiers) impart high and low temperature operability to a lubricating oil and permit it to remain shear stable at elevated temperatures and also exhibit acceptable viscosity or fluidity at low temperatures. Suitable compounds for use as viscosity modifiers are generally high molecular weight hydrocarbon polymers, including polyesters, and viscosity index improver dispersants, which function as dispersants as well as viscosity index improvers. Oil soluble viscosity modifying polymers generally have weight average molecular weights of from about 10,000 to 1,000,000, preferably 20,000 to 500,000, as determined by gel permeation chromatography or light scattering methods.

Representative examples of suitable viscosity modifiers are polyisobutylene, copolymers of ethylene and propylene, polymethacrylates, methacrylate copolymers, copolymers of an unsaturated dicarboxylic acid and a vinyl compound, interpolymers of styrene and acrylic esters, and partially hydrogenated copolymers of styrene/ isoprene, styrene/butadiene, and isoprene/butadiene, as well as the partially hydrogenated homopolymers of butadiene and isoprene.

Corrosion inhibitors, also known as anti-corrosive agents, reduce the degradation of the metallic parts contacted by the lubricating oil composition. Illustrative of corrosion inhibitors are phospho-sulphurized hydrocarbons and the products obtained by reaction of a phosphosulphurized hydrocarbon with an alkaline earth metal oxide or hydroxide, preferably in the presence of an alkylated phenol or of an alkylphenol thioester, and also preferably in the presence of carbon dioxide.

Phosphosulphurized hydrocarbons may be prepared by reacting a suitable hydrocarbon, for example, a terpene, a heavy petroleum fraction of a $C_2$ to $C_6$ olefin polymer such, for example, as polyisobutylene, with from 5 to 30 mass% of a sulphide of phosphorus for 1/2 to 15 hours, at a temperature in the range of about 65 to about 315 °C. Neutralization of the phosphosulphurized hydrocarbon may be effected in any suitable manner, for example, in the manner taught in U.S. Patent No. 1,969,324.

Oxidation inhibitors, or antioxidants, reduce the tendency of mineral oils to deteriorate in service, evidence of such deterioration being, for example, the production of varnish-like deposits on the metal surfaces and of sludge, and viscosity growth. Suitable oxidation inhibitors include alkaline earth metal salts of alkyl-phenolthioesters having preferably $C_5$ to $C_{12}$ alkyl side chains, e.g. calcium nonylphenyl sulphide; barium octylphenyl sulphide; dioctylphenylamine; phenylalpha-naphthylamine; and phosphosulphurized or sulphurized hydrocarbons.

Other oxidation inhibitors or antioxidants which may be used in lubricating oil compositions comprise oil-soluble copper compounds. The copper may be blended into the oil as any suitable oil-soluble copper compound. By oil-soluble it is meant that the compound is oil-soluble under normal blending conditions in the oil or additive package. The copper compound may be in the cuprous or cupric form. The copper may, for example, be in the form of a copper dihydrocarbyl thio-or dithio-phosphate. Alternatively, the copper may be added as the copper salt of a synthetic or natural carboxylic acid. Examples of suitable acids include $C_8$ to $C_{18}$ fatty acids, such, for example, as stearic or palmitic acid, but unsaturated acids such, for example, as oleic acid or branched carboxylic acids such, for example, as naphthenic acids of molecular weights of from about 200 to 500, or synthetic carboxylic acids, are preferred, because of the improved handling and solubility properties of the resulting copper carboxylates. Also useful are oil-soluble copper dithiocarbamates of the general formula $[R^1R^2NC(S)S]_zCu$ where z is 1 or 2, and $R^1$ and $R^2$ represent the same or different hydrocarbyl radicals containing from 1 to 18, and preferably 2 to 12, carbon atoms, and including radicals such, for example, as alkyl, alkenyl, aryl, aralkyl, alkaryl and cycloaliphatic radicals. Particularly preferred as $R^1$ and $R^2$ groups are alkyl groups having from 2 to 8 carbon atoms, for example, those listed earlier in this specification as preferred groups which $R^1$ and $R^2$ may represent. Copper sulphenates, phenates, and acetylacetonates may also be used.

Examples of further useful copper compounds are copper $Cu^I$ and/or $Cu^{II}$ salts of alkenyl succinic acids or anhydrides. The salts themselves may be basic, neutral or acidic. They may be formed by reacting (a) polyalkylene succinimides (having polymer groups of $\overline{M}_n$ of 700 to 5,000) derived from polyalkylene-polyamines, which have at least one free carboxylic acid group, with (b) a reactive metal compound. Suitable reactive metal compounds include those such, for example, as cupric or cuprous hydroxides, oxides, acetates, borates, and carbonates or basic copper carbonate.

Examples of these metal salts are Cu salts of polyisobutenyl succinic anhydride, and Cu salts of polyisobutenyl succinic acid. Preferably, the copper is in its divalent form, $Cu^{II}$. The preferred substrates are polyalkenyl succinic acids in which the alkenyl group has a number average molecular weight greater than about 700. The alkenyl group desirably has a $\overline{M}_n$ from about 900 to 1,400, and up to 2,500, with a $\overline{M}_n$ of about 950 being most preferred. Especially preferred is polyisobutylene succinic anhydride or acid. These materials may desirably be dissolved in a solvent, such as a mineral oil, and heated in the presence of a water solution (or slurry) of the metal-bearing material to a temperature of about 70 °C to about 200 °C. Temperatures of 100 °C to 140 °C are normally adequate. It may be necessary, depending upon the salt produced, not to allow the reaction mixture to remain at a temperature above about 140 °C for an extended period of time, e.g. longer than 5 hours, or decomposition of the salt may occur.

The copper antioxidants (e.g. Cu-polyisobutenyl succinic anhydride, Cu-oleate, or mixtures thereof) will generally be employed in an amount of from about 50 to 500 ppm by weight of the copper, in the final lubricating or fuel composition.

Friction modifiers and fuel economy agents which are compatible with the other ingredients of the final oil may also be included. Examples of such materials are glyceryl monoesters of higher fatty acids, for example, glyceryl mono-oleate, esters of long chain polycarboxylic acids with diols, for example, the butane diol ester of a dimerized unsaturated fatty acid, and oxazoline compounds.

Dispersants maintain oil-insoluble substances, e.g. resulting from wear or oxidation during use, in suspension in the fluid, thus preventing sludge flocculation and precipitation or deposition on metal parts. So-called ashless dispersants are organic materials which form substantially no ash on combustion, in contrast to metal-containing (and thus ash-forming) detergents. Suitable dispersants include, for example, derivatives of long chain hydrocarbon-substitutedcarboxylic acids in which the hydrocarbon groups contain 50 to 400 carbon atoms, examples of such derivatives being derivatives of high molecular weight hydrocarbyl-substituted succinic acid. As indicated above, such hydrocarbon-substituted carboxylic acids may be reacted with, for example, a nitrogen-containing compound, advantageously a polyalkylene polyamine, or with an ester. Such nitrogen-containing and ester dispersants are well known in the art. Particularly preferred dispersants are the reaction products of polyalkylene amines with alkenyl succinic anhydrides.

In general, suitable dispersants include oil soluble salts, amides, imides, oxazolines and esters, or mixtures thereof, of long chain hydrocarbon-substituted mono and dicarboxylic acids or their anhydrides;

long chain aliphatic hydrocarbons having a polyamine attached directly thereto; and Mannich condensation products formed by condensing about 1 molar proportion of a long chain substituted phenol with about 1 to 2.5 moles of formaldehyde and about 0.5 to 2 moles of a polyalkylene polyamine. In these dispersants long chain hydrocarbon groups are suitably derived from polymers of a $C_2$ to $C_5$ monoolefin, the polymers having a number average molecular weight of about 700 to about 5000.

As indicated above, a viscosity index improver dispersant functions both as a viscosity index improver and as a dispersant. Examples of viscosity index improver dispersants suitable for use in accordance with the invention include reaction products of amines, for example polyamines, with a hydrocarbyl-substitutedmono -or dicarboxylic acid in which the hydrocarbyl substituent comprises a chain of sufficient length to impart viscosity index improving properties to the compounds. In general, the viscosity index improver dispersant may be, for example, a polymer of a $C_4$ to $C_{24}$ unsaturated ester of vinyl alcohol or a $C_3$ to $C_{10}$ unsaturated mono- or di-carboxylic acid with an unsaturated nitrogen-containing monomer having 4 to 20 carbon atoms; a polymer of a $C_2$ to $C_{20}$ olefin with an unsaturated $C_3$ to $C_{10}$ mono- or di-carboxylic acid neutralised with an amine, hydroxyamine or an alcohol; or a polymer of ethylene with a $C_3$ to $C_{20}$ olefin further reacted either by grafting a $C_4$ to $C_{20}$ unsaturated nitrogen-containing monomer thereon or by grafting an unsaturated acid onto the polymer backbone and then reacting carboxylic acid groups of the grafted acid with an amine, hydroxy amine or alcohol.

Examples of dispersants and viscosity index improver dispersants may be found in European Patent Specification No. 24146 B, the disclosure of which is incorporated herein by reference.

As indicated earlier in the specification, detergents and metal rust inhibitors include the metal salts, which may be overbased, of sulphonic acids, alkyl phenols, sulphurised alkyl phenols, alkyl salicylates, naphthenates, and other oil-soluble mono- and dicarboxylic acids. Overbased metal sulphonates wherein the metal is selected from alkaline earth metals and magnesium are particularly suitable for use as detergents. Representative examples of detergents/rust inhibitors, and their methods of preparation, are given in European Specification No. 208 560 A.

Antiwear agents, as their name implies, reduce wear of metal parts. The ZDDPs mentioned earlier in this specification are very widely used as antiwear agents.

Pour point depressants, otherwise known as lube oil flow improvers, lower the temperature at which the fluid will flow or can be poured. Such additives are well known. Typical of those additives which improve the low temperature fluidity of the fluid are $C_8$ to $C_{18}$ dialkyl fumarate/vinyl acetate copolymers, polymethacrylates, and wax naphthalene. Foam control can be provided by an antifoamant of the polysiloxane type, for example, silicone oil or polydimethyl siloxane.

Additives prepared in accordance with the invention may where appropriate be used in lubricating oils other than engine oils.

Some of the above-mentioned additives can provide a multiplicity of effects; thus for example, a single additive may act as a dispersant-oxidation inhibitor. This approach is well known and need not be further elaborated herein.

Compositions when containing the above-mentioned additives are typically blended into the base oil in amounts which are effective to provide their normal function. Representative effective amounts of such additives for an automobile crankcase lubricant are:

| Additive | Mass % a.i.* (Broad) | Mass % a.i.* (Preferred) |
|---|---|---|
| Viscosity Modifier | 0.01-6 | 0.01-4 |
| Corrosion Inhibitor | 0.01-5 | 0.01-1.5 |
| Oxidation Inhibitor | 0.01-5 | 0.01-1.5 |
| Dispersant | 0.1-20 | 0.1-8 |
| Pour Point Depressant | 0.01-5 | 0.01-1.5 |
| Anti-Foaming Agent | 0.001-3 | 0.001-0.15 |
| Friction Modifier | 0.01-5 | 0.01-1.5 |
| Mineral or Synthetic Oil Base | Balance | Balance |

* Mass% active ingredient based on the final oil.

When a plurality of additives are employed it may be desirable, although not essential, to prepare additive concentrates comprising the additives (the concentrate being referred to herein as an additive package) whereby several additives can be added simultaneously to the base oil to form the lubricating oil composition. Dissolution of the additive concentrate into the lubricating oil may be facilitated by solvents

and by mixing accompanied with mild heating, but this is not essential. The concentrate or additive package will typically be formulated to contain the additive(s) in proper amounts to provide the desired concentration in the final formulation when the additive package is combined with a predetermined - amount of base lubricant. Thus, one or more additives can be added to small amounts of base oil or other compatible solvents along with other desirable additives to form additive packages containing active ingredients in an amount, based on the additive package, of, for example, from about 2.5 to about 90 mass%, and preferably from about 5 to about 75 mass%, and most preferably from about 8 to about 50 mass% by weight, additives in the appropriate proportions with the remainder being base oil.

Three forms of mixing apparatus and reactor suitable for use in the process of the invention will now be described, by way of example only, with reference to the accompanying drawings, in which:

Fig. 1 is a schematic longitudinal section through one form of reactor suitable for use in accordance with the invention;

Fig. 2 is a cross-section through the reactor shown in Fig. 1;

Fig. 3 is a schematic vertical section through a further form of reactor suitable for use in accordance with the invention;

Fig. 4 is a cross-section through the reactor shown in Fig. 3;

Fig. 5 is a schematic vertical section through a modification of the reactor shown in Fig. 3; and

Fig. 6 is a cross-section through the reactor shown in Fig. 5.

Referring now to the drawings, the reactor shown in Figs. 1 and 2 comprises a housing 1 having the general form of a right circular cylinder, the housing being arranged with its longitudinal axis substantially horizontal. The housing, which is closed at each end, is provided at one end (the left hand end as seen in the drawings) with inlets 2,3 for reactants to be treated in the reactor and at the other end with an outlet 4 for products of the reaction.

Within the housing, and coaxial therewith, is a shaft 5 which can be caused to rotate by drive means 6 situated outside the housing. A plurality of blades 7 extend radially outwards from the shaft, the end of each blade closer to the shaft being fixed to the shaft so that the blades rotate with the shaft. The shaft and blades thus form a rotor, which rotates relative to the housing, the housing remaining stationary during use of the reactor.

The outer extremities of the blades are adjacent to the inner surface of the housings, such that the said extremities and surface define a reaction region or zone. The width of the reaction region may be, for example, 0.2 to 10 mm.

In use of the reactor, the shaft is caused to rotate at high speed, for example, 10,000 rpm. Such rotation causes reactants introduced through the inlets to form a thin film on the inner wall of the housing, reaction between the reactants taking place in this thin film. Material within the housing travels along the inner surface of the housing until it reaches the outlet, through which it is removed for any further processing that may be necessary.

Because a thin film is formed on the inner wall of the housing, the reactants are treated in accordance with the process of the invention. A further advantage of reactors in which the reactants are reacted in a thin film on an outer housing is that the temperature of the materials forming the film can be controlled by supplying heat to, or withdrawing it from, the housing.

Figs. 3 and 4 show a reactor suitable for use in accordance with the invention. The reactor shown is particularly suitable for use when working on a laboratory scale, but may readily be adapted for larger scale operation.

The reactor comprises a casing 11 in the form of a right circular cylinder, the axis of the casing extending substantially vertically. The top and bottom of the casing are closed, the bottom being provided with inlets 12 and 13 for reactants and the top being provided with an outlet 14 for material that has passed through the reactor. When working on a large scale, it may be advantageous to use an arrangement in which the axis of the casing extends generally horizontally, as in Figs. 1 and 2.

Within the casing, and coaxial therewith, is a shaft 15, supported by a bearing, which can be caused to rotate by drive means (not shown) situated outside the casing. Attached to the lower end of the shaft is a rotor comprising a rotor disc 16 with an upwardly extending peripheral rotor flange 17. The disc is mounted on the shaft for rotational movement therewith, the shaft, disc and rotor flange thus forming a rotor. The disc is provided with three generally triangular apertures 18 therein, and the rotor flange has a plurality of circumferential openings 19 therein (most clearly seen in Fig. 4).

Also mounted in the casing is a stator comprising a stator disc 20 of larger diameter than the rotor disc 16, and two downwardly extending, circumferentially discontinuous, stator flanges 21 and 22, each of the said flanges being coaxial with the rotor flange, which extends upwardly between the two stator flanges. Each of the stator flanges comprises a plurality of wall portions 24 extending downwardly from the stator

disc such that there are openings 25 between the wall portions. As will be most clearly seen in Fig. 4, the outer surface of the rotor flange is adjacent to the inner surface of the outer stator flange 21, and the inner surface of the rotor flange is adjacent to the outer surface of the inner stator flange 22, so that the rotor and stator define two annular reaction regions, indicated by the reference numerals 26 and 27, in accordance with the invention.

The outer circumferential wall of the reactor shown in Figs. 3 and 4 may if desired be provided with a heating/cooling jacket (not shown).

In use of the reactor shown in Figs. 3 and 4, the shaft 15 is rotated and the reactants are introduced through the inlets 12 and 13. The pumping action of the rotor causes the reactants to be drawn through the apertures 18 in the rotor disc 16 and thus into the central region of the rotor/stator arrangement. The reactants are then forced by the rotor to travel outwards in a generally radial direction, such that they pass through the inner stator flange, the rotor flange, and the outer stator flange to the zone 28 between the outer stator flange and the casing. In passing through the rotor and stator flanges, the reactants react in the annular reaction regions 26 and 27.

Material in the zone 28 passes upwardly through the casing (the upper part of the casing may, if desired, be used as a post-reaction zone), and removed via the outlet 14.

Figs. 5 and 6 show a modification of the reactor shown in Figs. 3 and 4. In the reactor shown in Figs. 5 and 6, the reactor also includes two opposed radially extending blades 30, each of which has a downwardly extending portion 31 providing a surface 32 adjacent to the inner surface of the inner stator flange 22. As shown in Figs. 5 and 6, the blades 30 and the rotor disc 16 are both mounted on the same shaft, but if desired they could be mounted on different shafts, one extending through the top of the reactor, and one extending through the bottom.

Although the arrangement shown in Figs. 5 and 6 has one rotor flange and two stator flanges, arrangements with additional rotor and stator flanges may be used if desired, thus increasing the number of reaction regions in accordance with the invention. Thus, for example, in some circumstances an arrangement with two upwardly extending rotor flanges and three downwardly extending stator flanges has been found to be advantageous.

In the reactor shown in Figs. 5 and 6, the apertures 18 shown in Figs. 3 and 4 are replaced by a plurality of circular apertures 33.

The following Examples illustrate the invention. In the Examples, the reactor used in accordance with the invention was substantially as described with reference to Figs. 5 and 6 of the accompanying drawings, and had the following characteristics:

| | |
|---|---|
| Diameter of volume swept by rotor | = 40.5 mm |
| Gap between rotor and each stator | = 0.5 mm |
| Speed of rotation of rotor | = 10,000 rpm |

Example 1

A slurry of phosphorus pentasulphide ($P_2S_5$) (phosphorus content of 27.6 mass% and 4.8 mass% of free sulphur) and a mixture of $C_4$ and $C_5$ primary alcohols (35 mass% isobutanol, 25 mass% pentanol and 40 mass% 3-methyl-1-butanol) was fed continuously to the reactor through a coarse tube peristaltic pump at a flow rate of 10 g/min. 4.2 moles of alcohol were used for each mole of $P_2S_5$. The mixture in the reactor was maintained at a temperature of approximately 85 °C and the residence time in the reaction region of the reactor defined as:

$$\text{Residence time} = \frac{\text{volume of reaction region}}{\text{flow rate of reactants}}$$

was approximately 5 minutes.

A DDPA of constant quality and containing 11.8 mass% phosphorus and 0.01% sediments was obtained. Hydrogen sulphide, one mole of which was produced for each mole of $P_2S_5$ used, was removed from the system and scrubbed in a caustic medium. The rate of production of the DDPA was 600 g/h.

Example 2

The process was carried out as described in Example 1, but with the reactants continuously circulating through a system wherein the reactor used in accordance with the invention was coupled with a standard stirred tank reactor. After continuous circulation for 50 mins at 85°C a DPPA of constant quality having a phosphorus content of 11.9 mass% and containing less than 0.01% sediment was obtained. The rate of production of the DDPA was approximately 60 g/h.

Example 3

The procedure described in Example 1 was repeated replacing the alcohol mixture used in Example 1 with a mixture of $C_3$ and $C_6$ secondary alcohols (35 mass% isopropanol and 65 mass% methyl isobutyl carbinol) having an average molecular weight of 86.3, and maintaining the mixture in the reactor at approximately 80°C. A DDPA of constant quality and containing 11.3 mass% P and 0.01% sediment was obtained.

Example 4

The process was carried out as in Example 3, but with the reactants continuously circulating through a system as described in Example 2. After continuous circulation of 50 mins at 80°C, a DDPA of constant quality having a phosphorus content of 11.3 mass% and less than 0.01 mass% sediment was obtained.

Comparative Example 1

50 g of the reaction mixture used in Example 1 were charged into a discontinuous stirred tank reactor. After 4 hours a DDPA with 0.01 mass% sediment and a phosphorus content of 11.8 mass% was obtained. The rate of production of the DDPA was 12.5 g/h, as compared with a rate of 600 g/h for a 0.01% sediment product in Example 1 and 60 g/h for a <0.01% sediment product in Example 2.

Example 5

A slurry of ZnO in base oil (35.4 mass% ZnO based on the slurry) and a DDPA derived from a mixture of $C_4$ and $C_5$ primary alcohols and containing 12.0 mass% P were fed separately to the reactor, where the reactants were maintained at a temperature of 70°C. The DDPA was fed at a rate of 28.5 g/min and the ZnO slurry at a rate of 13 g/min. A raw ZDDP containing 0.25 mass% sediment and having a pH of 5 and a hydrolytic stability of greater than 60 mins was obtained. After filtration, a bright and clear product with a Zn/P ratio of 1.07, a basicity of 63 mg HCl/g, a Zn content of 8.59 mass% and a P content of 8.02 mass% was obtained.

Example 6

A DDPA as used in Example 5 was reacted with ZnO slurried in base oil (ZnO content 36.2 mass%, based on the slurry). The ZnO slurry was continuously circulated through the reactor used in accordance with the invention, which was maintained at 88°C, and a normal stirred tank reactor, which was maintained at 85°C, while the DDPA was fed separately to the reactor used according to the invention. An amount of DDPA corresponding to a Zn/P ratio of 1.08 was charged over a period of 30 minutes. After all the DDPA had been added, the mixture was circulated through the system for 50 minutes.

A raw ZDDP with 0.01 mass% sediment, a pH of 5.0 and hydrolytic stability of greater than 60 minutes was obtained. The ZDDP did not require filtration and, after nitrogen stripping at 80°C, was clear (40 NTU haze) as compared with raw ZDDP. The product had a Zn/P ratio of 1.08, a basicity of 29 mg HCl/g, a Zn content of 8.59 mass% and a P content of 7.98 mass%.

Example 7

A slurry of ZnO in base oil (35.5 mass% of ZnO based on the slurry) and a DDPA derived from a mixture of sec-butyl alcohol and iso-octyl alcohol (85/15 by mass) and containing 11.8% P were fed separately to the reactor, where the reactants were maintained at a temperature of 75°C. The DDPA was fed at a rate of 26.5 g/min and the ZnO slurry at a rate of 14.5 g/min. A raw ZDDP containing 1.0 mass%

sediment and having a pH of 4.8 and a hydrolytic stability of 10 minutes was obtained.

The raw ZDDP was transferred to a stirred tank reactor and maintained at 75°C for 60 minutes. A stable product was obtained containing 0.2% sediment and having a pH of 5.9. This product was filtered to yield a final product having a Zn/P ratio of 1.10:1, a basicity of 55 mg HCl/g, a Zn content of 8.71% and a P content of 7.95%.

Example 8

A system as described in Example 6 was used, both reactors being maintained at a temperature of 85°C. 2.5 mass% of the zinc was added as Zn acetate. The DDPA was the same as that used in Example 7, and an amount of the DDPA corresponding to a Zn/P ratio of 1.11:1 was introduced in 40 mins. After all the DDPA had been added, the mixture was circulated through the system for 30 minutes.

The procedure was repeated under the same conditions, but without the use of the promoter (that is, the zinc acetate).

The data obtained are summarised as follows:

| | With Promoter | Without Promoter |
|---|---|---|
| DDPA Addition time (mins) | 40 | 60 |
| pH | 5.8 | 4.8 |
| Stability (mins) | >60 | 20 |
| Mass% Sediment | 0.05 | 0.3 |
| Zn/P Ratio | 1.10 | 1.05 |
| Basicity (mg HCl/g) | 66 | not measured |

It will be seen that in this particular case higher stability and much lower sediments were obtained when a promoter was used. In addition, the use of the promoter made it possible to add the DDPA over a shorter period. When a promoter was used, a filtered product with a Zn/P ratio which is substantially the same as that in the material charged to the reactor was obtained.

Example 9

The procedure described in Example 8 was repeated, using the same promoter as in Example 8. The ZnO slurry used had a ZnO content of 45 mass%, and the DDPA was based on 2-ethylhexyl alcohol, and had a P content of 8.4 mass%.

The product had a pH of 5.8, a sediment level of 0.01 mass%, a stability of greater than 60 minutes, a Zn/P ratio of 1.09:1, and a basicity (mg HCl/g) of 63.

## Claims

1. A process for the manufacture of a compound of the general formula

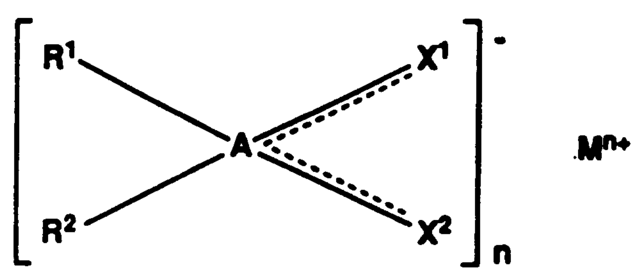

(I)

wherein A represents an -O-P-O- or an $>$N-C group;
each of $X^1$ and $X^2$, which may be the same or different, represents O or S;
each of $R^1$ and $R^2$, which may be the same or different, represents a hydrocarbyl radical,
M represents H, a metal ion, or an ammonium group; and
n represents an integer equal to the valency of M,

with the proviso that, when A represents an >N-C group, both $X^1$ and $X^2$ represent S, in which process a first reactant is present in a different phase from a second reactant and the said reactants are reacted in a region defined by a surface and at least one member adjacent to the surface, there being relative movement between the surface and the member(s) such that the reactants are caused to move relative to the surface.

**2.** A process as claimed in claim 1, wherein the region is defined by adjacent surfaces between which there is relative movement such that the reactants are caused to move relative to one or both of the surfaces.

**3.** A process for the manufacture of a compound of the general formula

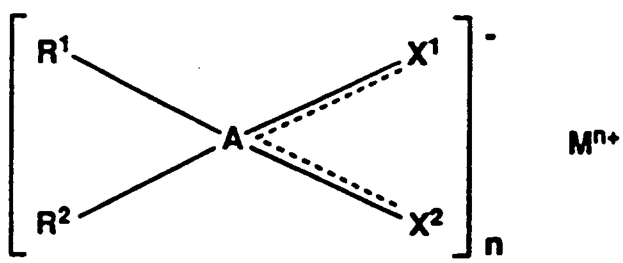

(I)

wherein A represents an -O-P-O- or an >N-C group;
each of $X^1$ and $X^2$, which may be the same or different, represents O or S;
each of $R^1$ and $R^2$, which may be the same or different, represents a hydrocarbyl radical,
M represents H, a metal ion, or an ammonium group; and
n represents an integer equal to the valency of M,
with the proviso that, when A represents an >N-C group, both $X^1$ and $X^2$ represent S,
in which process a first reactant is present in a different phase from a second reactant and the reactants are reacted in a thin film on a surface over which they move, the average velocity (taken across the thickness of the film) of a mixture of the reactants in a direction parallel to the surface being at least 1 m/sec.

**4.** A process as claimed in claim 3, wherein the thin film is formed between the surface and at least one member adjacent to the surface, there being relative movement between the surface and the member(s).

**5.** A process as claimed in claim 3, wherein the thin film is formed between adjacent surfaces between which there is relative movement.

**6.** A process as claimed in any one of claims 1, 2, 4 and 5, wherein the surface and the member(s), or the two surfaces, are separated by a distance of 0.1 to 10 mm.

**7.** A process as claimed in any one of claims 2, 5 and 6, wherein one of the surfaces is a surface of an inner body and the other surface is a surface of an outer housing, one of the inner body and the outer housing being a rotor, and the other being a stator.

**8.** A process as claimed in claim 7, wherein the inner body is a rotor and, in use, has a peripheral speed of 20 to 30 m/sec.

**9.** A process as claimed in claim 7 or claim 8, wherein the inner body and/or the outer body has one or more apertures or discontinuities therein.

**10.** A process as claimed in any one of claims 7 to 9, wherein apparatus comprising a plurality of rotors and/or stators is used.

**11.** A process as claimed in any one of claims 7 to 10, wherein the rotor(s) and stator(s) form part of a reactor which is provided with one or more inlets and one or more outlets, and the rotor(s), stator(s), inlet(s) and outlet(s) are so arranged that the reactants can be introduced in an axial direction to the centre of the rotor/stator arrangement and can pass radially through at least one rotor or stator before leaving the reactor.

**12.** A process as claimed in any one of claims 1 to 11, which is carried out in a continuous manner.

**13.** A process as claimed in claims 1 to 12, wherein at least 75% of each of the reactants is treated in a reaction region as specified in claim 1 or a thin film as specified in claim 3.

**14.** A process as claimed in any one of claims 1 to 13, in which the reactants and/or reaction product(s) are also subjected to a soaking step.

**15.** A process as claimed in any one of claims 1 to 14, wherein the compound has the general formula [-$(R^1O)(R^2O)P(S)(O)]_n^-$ $M^{n+}$, wherein $R^1$, $R^2$, M and n have the meanings given in claim 1.

**16.** A process as claimed in any one of claims 1 to 14, wherein the compound has the general formula [-$(R^1O)(R^2O)P(S)S]_n^-$ $M^{n+}$, wherein $R^1$, $R^2$, M and n have the meanings given in claim 1.

**17.** A process as claimed in any one of claims 1 to 14, wherein the compound has the general formula $[R^1R^2NC(S)S]_n^-$ $M^{n+}$, wherein $R^1$, $R^2$, M and n have the meanings given in claim 1.

**18.** A process as claimed in any one of claims 1 to 17, wherein the total number of carbon atoms in $R^1$ and $R^2$ is sufficient to impart oil-solubility to the compound.

**19.** A process as claimed in any one of claims 1 to 18, wherein M represents a Group Ia metal, a Group IIa metal, aluminium, tin, lead, molybdenum, titanium, manganese, cobalt, nickel, copper, cadmium, antimony, or zinc.

**20.** A process as claimed in claim 19, wherein M represents zinc or copper.

**21.** A process as claimed in any one of claims 1 to 20, wherein each of the first and second reactants, independently, is present in the reaction mixture as a liquid or solid.

**22.** A compound as defined in claim 1, whenever prepared by a process as claimed in any one of claims 1 to 21.

**23.** The use of apparatus comprising an outer housing and a rotor within the housing, the housing and the rotor having opposed adjacent surfaces defining a reaction region, in the preparation of a compound as defined in claim 1.

**24.** The use of apparatus comprising a reaction region defined by a surface and at least one member adjacent to the surface, and means for causing relative movement between the surface and the member(s), for reducing sediments in a process for the manufacture of a compound of the general formula given in claim 1.

**25.** The use of apparatus comprising means for causing reactants to move in a thin film over a surface, for reducing sediments in a process for the manufacture of a compound of the general formula given in claim 1.

**Patentansprüche**

1. Verfahren zur Herstellung einer Verbindung mit der allgemeinen Formel

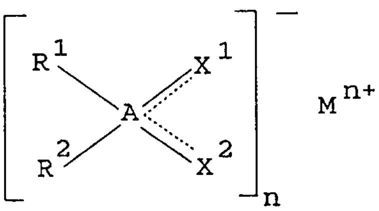

(I)

in der A eine -O-P-O- oder eine >N-C Gruppe bedeutet,
$X^1$ und $X^2$, die gleich oder verschieden sein können, jeweils O oder S bedeuten,
$R^1$ und $R^2$, die gleich oder verschieden sein können, jeweils einen Kohlenwasserstoffrest bedeuten,
M H, ein Metallion oder eine Ammoniumgruppe bedeutet,
n eine ganze Zahl gleich der Wertigkeit von M mit der Maßgabe bedeutet, daß, wenn A eine >N-C Gruppe bedeutet, sowohl $X^1$ als auch $X^2$ S bedeuten,
wobei bei dem Verfahren ein erster Reaktant in einer anderen Phase als ein zweiter Reaktant vorliegt und diese Reaktanten in einem Bereich umgesetzt werden, der durch eine Oberfläche und mindestens einen sich in Nachbarschaft zu der Oberfläche befindlichen Beteiligten festgelegt ist, wobei es eine relative Bewegung zwischen der Oberfläche und dem/den Beteiligten gibt, sodaß die Reaktanten veranlaßt werden, sich relativ zu der Oberfläche zu bewegen.

2. Verfahren nach Anspruch 1, bei dem der Bereich durch benachbarte Oberflächen festgelegt ist, zwischen denen es eine relative Bewegung gibt, sodaß die Reaktanten veranlaßt werden, sich relativ zu einer oder beiden Oberflächen zu bewegen.

3. Verfahren zur Herstellung einer Verbindung mit der allgemeinen Formel

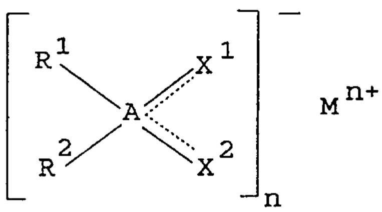

(I)

in der A eine -O-P-O- oder eine >N-C Gruppe bedeutet, $X^1$ und $X^2$, die gleich oder verschieden sein können, jeweils O oder S bedeuten,
$R^1$ und $R^2$, die gleich oder verschieden sein können, jeweils einen Kohlenwasserstoffrest bedeuten,
M H, ein Metallion oder eine Ammoniumgruppe bedeutet,
n eine ganze Zahl gleich der Wertigkeit von M mit der Maßgabe bedeutet, daß, wenn A eine >N-C Gruppe bedeutet, sowohl $X^1$ als auch $X^2$ S bedeuten,
wobei bei dem Verfahren ein erster Reaktant in einer anderen Phase als ein zweiter Reaktant vorliegt und diese Reaktanten in einem dünnen Film auf der Oberfläche umgesetzt werden, über die sie sich bewegen, wobei die durchschnittliche Geschwindigkeit (genommen über die Dicke des Films) einer Mischung der Reaktanten in einer Richtung parallel zu der Oberfläche mindestens 1 m/s beträgt.

4. Verfahren nach Anspruch 3, bei dem der dünne Film zwischen der Oberfläche und mindestens einem sich in Nachbarschaft zu der Oberfläche befindenden Beteiligten gebildet wird, wobei es eine relative Bewegung zwischen der Oberfläche und dem/den Beteiligten gibt.

5. Verfahren nach Anspruch 3, bei dem der dünne Film zwischen benachbarten Oberflächen gebildet wird, zwischen denen es eine relative Bewegung gibt.

6. Verfahren nach einem der Ansprüche 1, 2, 4 und 5, bei dem die Oberfläche und der Beteiligte/die Beteiligten oder die beiden Oberflächen durch einen Anstand von 0,1 bis 10 mm voneinander getrennt sind.

7. Verfahren nach einem der Ansprüche 2, 5 und 6, bei dem eine der Oberflächen die Oberfläche eines Innenkörpers und die andere Oberfläche die Oberfläche eines Außengehäuses ist, wobei einer von dem Innenkörper und dem Außengehäuse ein Rotor und der andere ein Stator ist.

8. Verfahren nach Anspruch 7, bei dem der Innenkörper ein Rotor ist und im Betrieb eine Umfangsgeschwindigkeit von 20 bis 30 m/s hat.

9. Verfahren nach Anspruch 7 oder Anspruch 8, bei dem der Innenkörper und/oder der Außenkörper eine oder mehrere Öffnungen oder Unstetigkeiten aufweist.

10. Verfahren nach einem der Ansprüche 7 bis 9, bei dem eine Apparatur mit mehreren Rotoren und/oder Statoren verwendet wird.

11. Verfahren nach einem der Ansprüche 7 bis 10, bei dem der Rotor/die Rotoren und der Stator/die Statoren einen Teil des Reaktors bilden, der mit einem oder mehreren Einlässen und einem oder mehreren Auslässen versehen ist, und der Rotor/die Rotoren, der Stator/die Statoren, der Einlaß/die Einlässe und der Auslaß/die Auslässe so angeordnet sind, daß die Reaktanten in einer axialen Richtung in die Mitte der Rotor/Stator-Anordnung eingebracht werden können und radial mindestens einen Rotor oder Stator passieren, bevor sie den Reaktor verlassen.

12. Verfahren nach einem der Ansprüche 1 bis 11, das kontinuierlich durchgeführt wird.

13. Verfahren nach Anspruch 1 bis 12, bei dem mindestens 75 % von jedem der Reaktanten in einem Reaktionsbereich gemäß Anspruch 1 oder einem dünnen Film gemäß Anspruch 3 behandelt werden.

14. Verfahren nach einem der Ansprüche 1 bis 13, bei dem die Reaktanten und/oder das Reaktionsprodukt/die Reaktionsprodukte außerdem einer Einweichstufe unterworfen werden.

15. Verfahren nach einem der Ansprüche 1 bis 14, bei dem die Verbindung die allgemeine Formel $[(R^1O)(R^2O)P(S)(O)]^-{}_n\ M^{n+}$ aufweist, in der $R^1$, $R^2$, M und n die in Anspruch 1 angegebenen Bedeutungen haben.

16. Verfahren nach einem der Ansprüche 1 bis 14, bei dem die Verbindung die allgemeine Formel $[(R^1O)(R^2O)P(S)S]^-{}_n\ M^{n+}$ aufweist, in der $R^1$, $R^2$, M und n die in Anspruch 1 angegebenen Bedeutungen haben.

17. Verfahren nach einem der Ansprüche 1 bis 14, bei dem die Verbindung die allgemeine Formel $[R^1R^2NC(S)S]^-{}_n\ M^{n+}$ aufweist, in der $R^1$, $R^2$, M und n die in Anspruch 1 angegebenen Bedeutungen haben.

18. Verfahren nach einem der Ansprüche 1 bis 17, bei dem die Gesamtanzahl der Kohlenstoffatome in $R^1$ und $R^2$ ausreichend ist, um der Verbindung Öllöslichkeit zu verleihen.

19. Verfahren nach einem der Ansprüche 1 bis 18, bei dem M ein Gruppe Ia Metall, ein Gruppe IIa Metall, Aluminium, Zinn, Blei, Molybdän, Titan, Mangan, Kobalt, Nickel, Kupfer, Cadmium, Antimon oder Zink wiedergibt.

20. Verfahren nach Anspruch 19, bei dem M Zink oder Kupfer wiedergibt.

21. Verfahren nach einem der Ansprüche 1 bis 20, bei dem jeder der ersten und zweiten Reaktanten unabhängig in der Reaktionsmischung als Flüssigkeit oder Feststoff vorhanden ist.

**22.** Verbindung wie in Anspruch 1 definiert, die nach einem Verfahren gemäß einem der Ansprüche 1 bis 21 hergestellt ist.

**23.** Verwendung einer Apparatur, die ein Außengehäuse und einen Rotor in dem Gehäuse umfaßt, wobei das Gehäuse und der Rotor sich gegenüberliegende benachbarte Oberflächen aufweisen, die einen Reaktionsbereich festlegen, zur Herstellung einer Verbindung wie in Anspruch 1 definiert.

**24.** Verwendung einer Apparatur, die einen Reaktionsbereich, welcher durch eine Oberfläche und mindestens einen sich in Nachbarschaft zu der Oberfläche befindenden Beteiligten festgelegt wird, und Mittel zum Veranlassen einer relativen Bewegung zwischen der Oberfläche und dem/den Beteiligten umfaßt, zur Verringerung der Sedimente in einem Verfahren zur Herstellung einer Verbindung mit der in Anspruch 1 angegebenen allgemeinen Formel.

**25.** Verwendung einer Apparatur, die Mittel umfaßt, um die Reaktanten zu veranlassen, sich in einem dünnen Film über die Oberfläche zu bewegen, zur Verringerung der Sedimente in einem Verfahren zur Herstellung einer Verbindung mit der in Anspruch 1 angegebenen allgemeinen Formel.

**Revendications**

**1.** Procédé de production d'un composé de formule générale

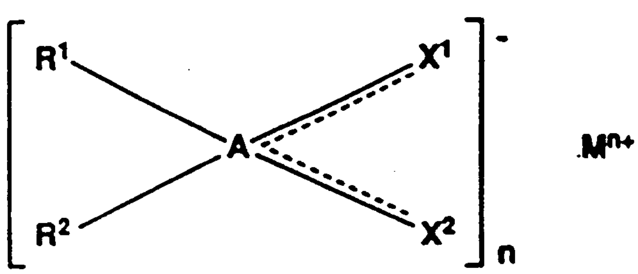

(I)

dans laquelle A représente un groupe -O-P-O- ou un groupe $>$N-C ;

chacun des groupes $X^1$ et $X^2$, qui peuvent être identiques ou différents, représente O ou S ;

chacun des groupes $R^1$ et $R^2$, qui peuvent être identiques ou différents, représente un radical hydrocarbyle,

M représente H, un ion métallique ou un groupe ammonium ; et

n représente un nombre entier égal à la valence de M,

sous réserve que, lorsque A représente un groupe $>$N-C, $X^1$ et $X^2$ représentent l'un et l'autre S,

procédé dans lequel le premier corps réactionnel est présent dans une phase différente de celle d'un second corps réactionnel et lesdits corps réactionnels sont amenés à réagir dans une région définie par une surface et au moins un élément adjacent à la surface, un mouvement relatif existant entre la surface et le ou les éléments de telle sorte que les corps réactionnels soient amenés à se déplacer par rapport à la surface.

**2.** Procédé suivant la revendication 1, dans lequel la région est définie par des surfaces adjacentes entre lesquelles il existe un mouvement relatif de telle sorte que les corps réactionnels soient amenés à se déplacer par rapport à une des surfaces ou bien aux deux surfaces.

**3.** Procédé de production d'un composé de formule générale

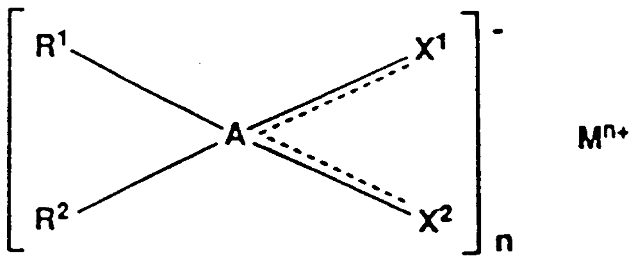

(I)

dans laquelle A représente un groupe-O-P-O- ou un groupe $>$N-C ;

chacun des groupes $X^1$ et $X^2$, qui peuvent être identiques ou différents, représente O ou S ;

chacun des groupes $R^1$ et $R^2$, qui peuvent être identiques ou différents, représente un radical hydrocarbyle,

M représente H, un ion métallique ou un groupe ammonium ; et

n représente un nombre entier égal à la valence de M,

sous réserve que, lorsque A représente un groupe $>$N-C, $X^1$ et $X^2$ représentent l'un et l'autre S,

procédé dans lequel le premier corps réactionnel est présent dans une phase différente de celle d'un second corps réactionnel et lesdits corps réactionnels sont amenés à réagir sous forme d'un film mince sur une surface sur laquelle ils se déplacent, la vitesse moyenne (déterminée à travers l'épaisseur du film) d'un mélange des corps réactionnels dans une direction parallèle à la surface étant au moins égale à 1 m/s.

**4.** Procédé suivant la revendication 3, dans lequel le film mince est formé entre la surface et au moins un élément adjacent à la surface, un mouvement relatif existant entre la surface et le ou les éléments.

**5.** Procédé suivant la revendication 3, dans lequel le film mince est formé entre des surfaces adjacentes entre lesquelles il existe un mouvement relatif.

**6.** Procédé suivant l'une quelconque des revendications 1, 2, 4 et 5, dans lequel la surface et le ou les éléments, ou bien les deux surfaces, sont séparés par une distance de 0,1 à 10 mm.

**7.** Procédé suivant l'une quelconque des revendications 2, 5 et 6, dans lequel une des surfaces est une surface d'un corps intérieur et l'autre surface est une surface d'un boîtier extérieur, un des éléments choisis entre le corps intérieur et le boîtier extérieur étant un rotor et l'autre étant un stator.

**8.** Procédé suivant la revendication 7, dans lequel le corps intérieur est un rotor et qui, lors de l'utilisation, possède une vitesse périphérique de 20 à 30 m/s.

**9.** Procédé suivant la revendication 7 ou la revendication 8, dans lequel le corps intérieur et/ou le boîtier extérieur renferment une ou plusieurs ouvertures ou discontinuités.

**10.** Procédé suivant l'une quelconque des revendications 7 à 9, dans lequel un appareil comprenant une pluralité de rotors et/ou de stators est utilisé.

**11.** Procédé suivant l'une quelconque des revendications 7 à 10, dans lequel le ou les rotors et le ou les stators font partie d'un réacteur qui est muni d'un ou plusieurs orifices d'admission et d'un ou plusieurs orifices de sortie, et le ou les rotors, le ou les stators, le ou les orifices d'émission et le ou les orifices de sortie sont disposés de telle sorte que les corps réactionnels puissent être introduits dans une direction axiale par rapport au centre de la structure de rotors/stators et puissent passer radialement à travers au moins un rotor ou stator avant de quitter le réacteur.

**12.** Procédé suivant l'une quelconque des revendications 1 à 11, qui est mis en oeuvre de manière continue.

**13.** Procédé suivant les revendications 1 à 12, dans lequel une quantité d'au moins 75 % de chacun des corps réactionnels est traitée dans une zone réactionnelle spécifiée dans la revendication 1 ou un film mince spécifié dans la revendication 3.

**14.** Procédé suivant l'une quelconque des revendications 1 à 13, dans lequel les corps réactionnels et/ou le ou les produits de réaction sont également soumis à une étape d'imprégnation.

**15.** Procédé suivant l'une quelconque des revendications 1 à 14, dans lequel le composé répond à la formule générale $[(R^1O)(R^2O)P(S)(O)]^-_n\ M^{n+}$, dans laquelle $R^1$, $R^2$, M et n répondent aux définitions mentionnées dans la revendication 1.

**16.** Procédé suivant l'une quelconque des revendications 1 à 14, dans lequel le composé répond à la formule générale $[(R^1O)(R^2O)P(S)(S]^-_nM^{n+}$, dans laquelle $R^1$, $R^2$, M et n répondent aux définitions mentionnées dans la revendication 1.

**17.** Procédé suivant l'une quelconque des revendications 1 à 14, dans lequel le composé répond à la formule générale $[R^1R^2NC(S)S]^-_nM^{n+}$, dans laquelle $R^1$, $R^2$, M et n répondent aux définitions mentionnées dans la revendication 1.

**18.** Procédé suivant l'une quelconque des revendications 1 à 17, dans lequel le nombre total d'atomes de carbone dans $R^1$ et $R^2$ est suffisant pour conférer une solubilité dans l'huile au composé.

**19.** Procédé suivant l'une quelconque des revendications 1 à 18, dans lequel M représente un métal du Groupe Ia, un métal du Groupe IIa, l'aluminium, l'étain, le plomb, le molybdène, le titane, le manganèse, le cobalt, le nickel, le cuivre, le cadmium, l'antimoine ou le zinc.

**20.** Procédé suivant la revendication 19, dans lequel M représente le zinc ou le cuivre.

**21.** Procédé suivant l'une quelconque des revendications 1 à 20, dans lequel chacun des premier et second corps réactionnels, indépendamment, est présent dans le mélange réactionnel sous forme d'un liquide ou d'une substance solide.

**22.** Composé suivant la revendication 1, préparé par un procédé suivant l'une quelconque des revendications 1 à 21.

**23.** Utilisation d'un appareil comprenant un boîtier extérieur et un rotor à l'intérieur du boîtier, le boîtier et le rotor possédant des surfaces adjacentes opposées définissant une zone réactionnelle, dans la préparation d'un composé répondant à la définition suivant la revendication 1.

**24.** Utilisation d'un appareil comprenant une zone réactionnelle définie par une surface et au moins un élément adjacent à la surface, et un moyen pour provoquer un mouvement relatif entre la surface et le ou les éléments, pour réduire la teneur en sédiments dans un procédé de production d'un composé répondant à la formule générale mentionnée dans la revendication 1.

**25.** Utilisation d'un appareil comprenant un moyen pour provoquer le déplacement des corps réactionnels dans un film mince sur une surface, pour réduire la teneur en sédiments dans un procédé de production d'un composé répondant à la formule générale mentionnée dans la revendication 1.

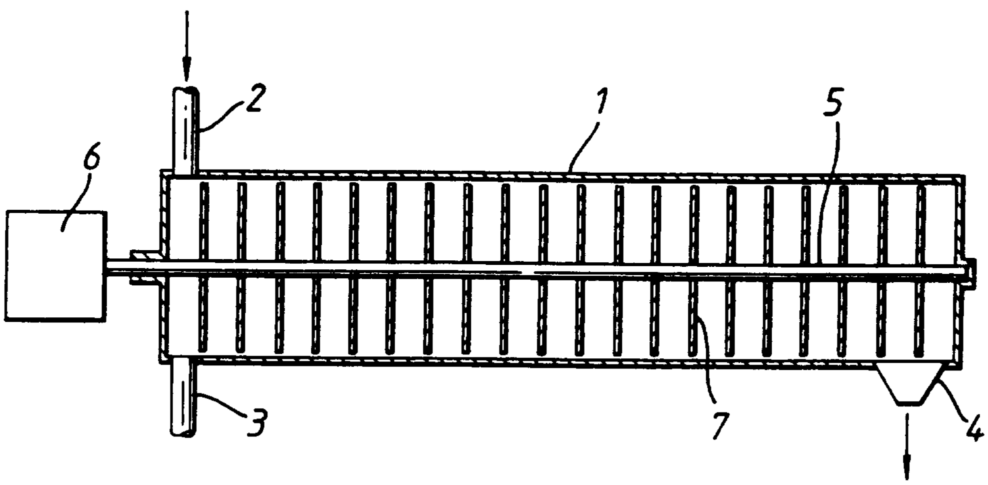


Fig.1.

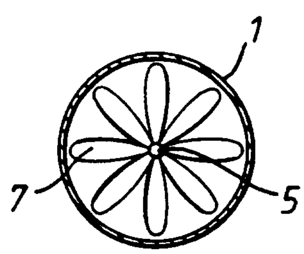


Fig.2.

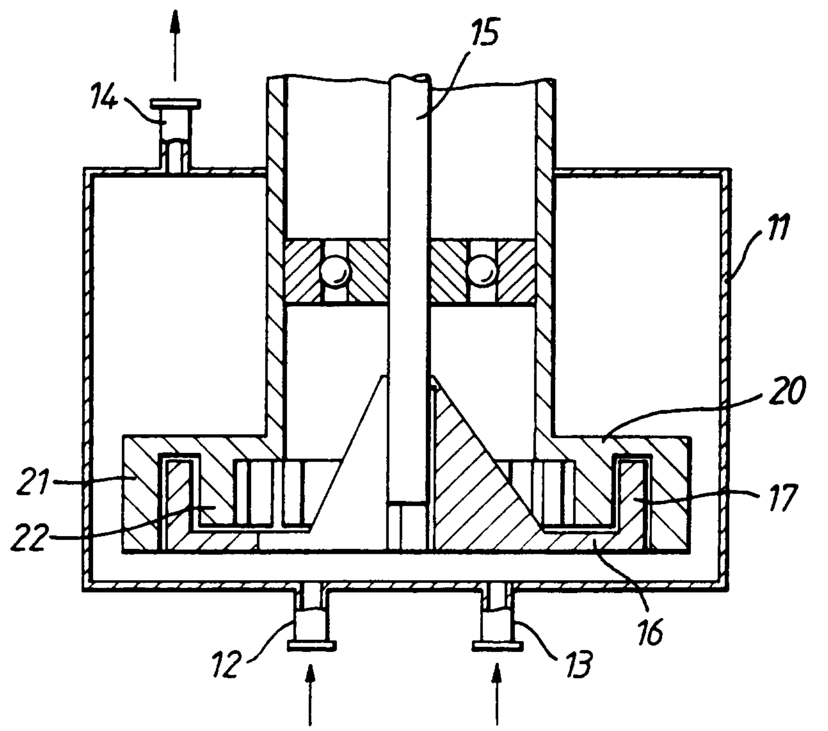


Fig.3.

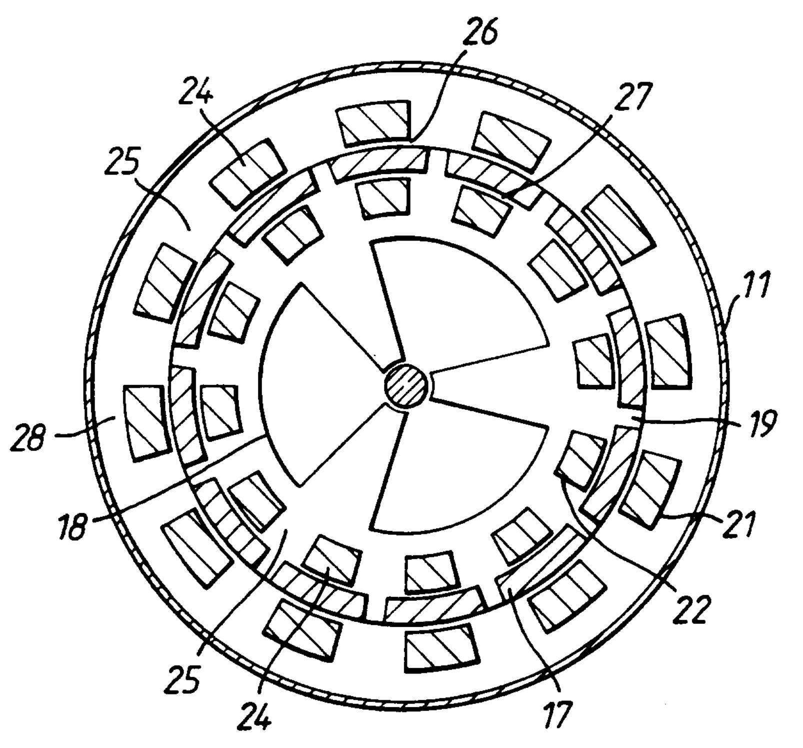


Fig.4.

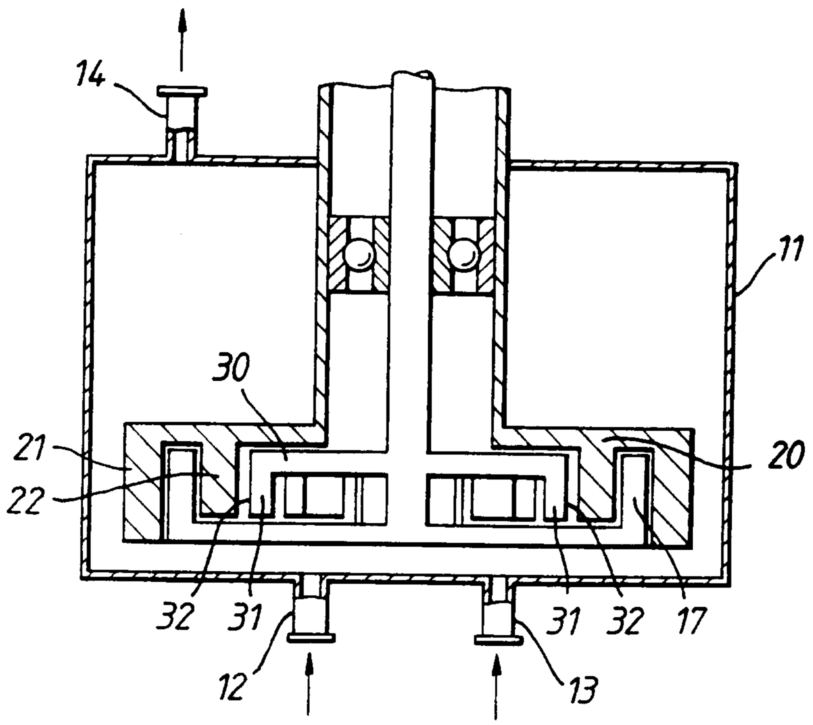
14
11
30
21
22
20
32
31
31
32
17
12
13

Fig.5.

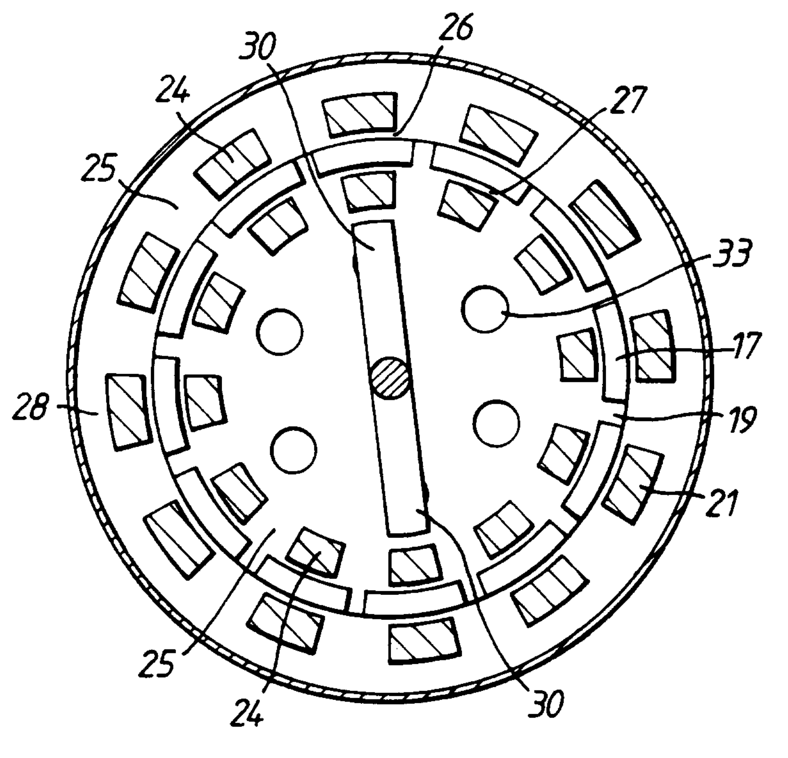
26
30
24
27
25
33
17
28
19
21
25
24
30

Fig.6.